# EUROPEAN PATENT SPECIFICATION

(11) **EP 1 339 413 B1**
(45) Date of publication and mention of the grant of the patent: **07.10.2009**
(21) Application number: 01983308.6
(22) Date of filing: 14.11.2001
(51) Int. Cl.: A61K 31/662, A61K 31/355, A61Q 19/08, A61Q 5/00, A61Q 1/00, A61Q 11/00, C07F 9/141, A61P 17/16, A61K 31/661, A61K 31/6615

(54) **Compositions comprising complexes of phosphate derivatives of tocopherol**
Zusammensetzungen umfassend Komplexe von Tocopherolphosphatderivaten
Compositions comprenant des complexes des dérivés de phosphate du tocophérol

(30) Priority: 14.11.2000 US 247997 P
(43) Date of publication of application: 03.09.2003
(73) Proprietor: Vital Health Sciences Pty Ltd., Melbourne, VIC 3000 (AU)
(72) Inventor: WEST, Simon, Michael, Williamstown, VIC 3016 (AU); VERDICCHIO, Robert, J., Succasunna, NJ 07876 (US); KANNAR, David, Belgrave South, VIC 3150 (AU)
(74) Representative: Berryman, Natalia Grace
(86) International application number: PCT/AU2001/001476
(87) International publication number: WO 2002/040034

(56) References cited:
- EP-A- 0 430 336
- EP-A- 0 845 216
- EP-A- 0 965 328
- WO-A-96/17852
- US-A- 5 387 579
- US-A- 6 046 181
- DATABASE WPI Section Ch, Week 199706 Derwent Publications Ltd., London, GB; Class B05, AN 1997-061803 XP002301027 & JP 08 311085 A (KAO CORP) 26 November 1996 (1996-11-26)
- DATABASE WPI Week 199807, Derwent Publications Ltd., London, GB; Class B02, AN 1998-071819, XP002968243 & JP 9 309 813 A (NONOGAWA SHOJI KK) 02 December 1997
- DATABASE CA [Online] XP002968707 Retrieved from STN Database accession no. 135:226884 & JP 2000 058632 A (NOF CORP) 03 March 2000
- DATABASE WPI Week 198416, Derwent Publications Ltd., London, GB; Class B03, AN 1984-098344, XP002058869 & JP 59 044 375 A (SENJU SEIYAKU KK) 12 March 1984

## Description

### Field of the invention

The invention relates to complexes of phosphate derivatives of tocopherol.

### Background of the invention

In this specification, where a document, act or item of knowledge is referred to or discussed, this reference or discussion is not to be taken as an admission that the document, act or item of knowledge was at the priority date:
(a) part of common general knowledge; or
(b) known to be relevant to an attempt to solve any problem with which this specification is concerned.

Over the past century, quantitative structure activity relationships (QSAR) have evolved and predominate in medicinal chemistry research programs. QSAR methods generate mathematical models to describe the biological function of drug formulations. Deriving a mathematical description of biological activity is characterized by two assumptions with respect to the relationship between the chemical structure and the biological potency of a compound. The first is that one can transform the chemical structure of a compound into numerical descriptors relevant to biological activity of a compound. The second establishes a quantitative relationship between these mathematical descriptors and potential biological activity.

The mathematical descriptors are usually either physiochemical, such as p*K*a or partition coefficient, or substructural, such as the presence or absence of functional groups, e.g. CO₂R or SH, and assist the formulating chemist to improve the solubility of the biologically active compound.

This is recognized to revolve around fundamental strategies aimed to increase solubility and dissolution rate of drugs derived from dosage forms. Theoretically, these strategies make the drug more available for absorption, and involve techniques such as co-solvent addition, solid state manipulation and pro-drug modification.

### Lipids as carriers

A number of drugs are more lipid soluble rather than water soluble, therefore lipids have been the carrier of choice for such drugs. Lipids are selected as drug vehicles based on their digestibility. Surfactant and co-solvent addition can facilitate digestion by increasing solubilization within the intestine and formation of chylomicrons/VLDL by the enterocyte to improve lymphatic transport.

Lipid-based formulations, in particular self-emulsifying drug delivery systems (SEDDS) and self micro-emulsifying drug delivery systems (SMEDDS) which utilize isotropic mixtures of triglyceride oil, non-surfactants and drugs, have been shown to overcome some of the barriers resulting in improved absorption characteristics and more reproducible plasma profiles of selected drugs.

SEDDS and SMEDDS can be filed into either soft or hard gelatine capsules, allowing rapid emulsification following release of the capsule contents and exposure to gentle agitation in an aqueous medium. Following emulsification, the fine oil droplets (< 5 µm in diameter) empty rapidly from the stomach and promote wide distribution of the lipophilic drug throughout the gastrointestinal tract. This fine droplet distribution increases surface area for the drug to partition into the intestine and should theoretically improve absorption.

### Derivatisation

Another strategy to improve solubility is to derivatise the compound, also known as forming pro-drugs. A number of undesirable properties may preclude the use of potentially valuable drugs in, clinical practice. Derivatisation has long been recognized as an important means of increasing efficacy and bioavailability of such drugs. Pro-drugs may be of limited value unless the pro-drug displays adequate stability, solubility, permeability and capability to revert to the parent compound once absorbed into the systemic circulation.

For example, one earlier attempt to address this problem involved forming covalent bonds with sugars and polyalcohols. However, further problems were created as the additional substituent must then be removed before drug activity is regenerated For example, tocopherol polyethylene glycol succinate (TPGS) is being sold as a water soluble derivative of α-tocopherol. There are indications that this derivative is absorbed even when bile secretion is impaired. However, the issue of hydrolysis of the ester linkage to succinate and metabolism of the resulting polyethylene glycol 1000 does not seem to have been addressed. It has not been established if and when the ester group hydrolyses. If the ester group does not hydrolyse then the tocopherol is not released and cannot act on the body. If the ester is hydrolysed then the next issue is whether the body can metabolise the polyethylene glycol by-product and dispose of it. If the body cannot metabolise the by-product then there could be a build up of by-product leading to side-effects. The TPGS product is also inconvenient and difficult to utilize clinically.

### Limitations of current drug solubilisation strategies

Today, QSAR remains a useful tool to help discover, quantify and evaluate possibles biological activity. However, QSAR has been criticized for not being able to effectively generate descriptors for three dimensional features, such as hydrophobicity and some electronic effects of drug interaction including hydrogen bonding. QSAR is also known to be inadequate in relation to describing various biological processes including gastrointestinal absorption, distribution, metabolism and excretion.

Development of lipid formulation strategies has also been helpful but only based upon the assumption that important biologically active compounds are passively absorbed and providing a dissolution gradient will improve absorption. This assumption is flawed and does not account for the possibility of active absorption. This delivery strategy therefore remains limited and cannot account for the fact that even after optimal formulation, absorption of poorly soluble nutrients from food is higher.

While ester derivatisation and solubilistion in SEDDS are known to improve lymphatic transport by the notion of forming small lipidic artificial chylomicrons, the methods are inefficient and probably more important to permit metabolism, rather than increasing transport of intact lipidic microstructures recognisable by transfer proteins. The use of alternative historic formulation strategies may therefore even restrict clinical utility of α-tocopherol and result in reduced efficacy.

### Examples of the limitations of OSAR formulation approaches

Tocopherol (vitamin E) is a poorly absorbed, lipid soluble vitamin and chemically unstable due to oxidation of the phenolic group. The majority of natural tocopherol is currently extracted from soy oil distillate and presented as simple substituted esters - either succinate or acetate derivatives. While this is primarily undertaken to prevent oxidation of the phenolic group and enhance stability, derivatisation is also thought to improve lymphatic transport There have been a number of attempts to enhance α-tocopherol acetate lymphatic transport via lipid formulation approaches. However despite some improvement, the extent of α-tocopheryl ester absorption after oral supplement administration is still poor and subject to large inter-patient variation. In contrast, dietary intake of vitamin E may result in a rapid and parallel increase in the content of α-tocopherol in blood plasma and erythrocytes.

Other drugs and nutrients are also subject to poor and variable absorption properties following current oral formulation strategies including phenytoin, vitamin A and CoQ₁₀, suggesting that physio-chemical factors other than dispersion, digestion and solubilisation control their bioavailability.

### Transportation

In recent years it has become apparent that absorption across biological membranes of some pharmacologically actives compounds, e.g. drugs and nutrients (vitamin E, ubiquinone, etc.), and endogenously important compounds such as phospholipids may be the limiting factor for bioavailability. As suggested such biological processes are difficult to describe mathematically as they are often multidimensional. It is therefore proposed that gastrointestinal uptake and transport of many biologically active compounds is dependent on other transportation mechanisms.

Studies have shown that α-tocopherol, phosphate is an effective, antioxidant and capable of preventing hypoxanthine/xanthine oxidase induced oxidative damage. α-Tocopherol phosphate is more water soluble than tocopherol or its succinate esters. These studies indicate that α-tocopherol phosphate not only improves chylomicron formation but also improves tissue penetration.

The art of efficient drug delivery therefore requires that the drug be not only soluble in the aqueous biological medium but in an appropriate form to permit transport of either individual drug molecules or very small aggregates of the drug molecules. This aim may be difficult to realize with drugs that are lipid soluble and not significantly water soluble. Such drug molecules have hydrophobic regions that form large aggregates in the high dielectric constant water rich medium where transport occurs. As a result, there have been investigations to discover a drug delivery system which increases the water solubility of the drugs.

international patent application no. PCT/AU00/00452 (WO 00/69865) teaches the formation of phosphorylated complex alcohols in conditions which preserve the complex alcohols. These complex alcohols include hormones, phytosterols, tocopherols (chromans), vitamin K1 and other oil-soluble vitamins and dietary supplements as well as drug compounds such as amoxycillin. These phosphorylated complex alcohols are more water soluble than the complex alcohols themselves, but it is desirable to achieve a yet higher level of bioavailability.

In summary, effective delivery of poorly water soluble compounds should not only provide delivery to the intestinal wall but also promote transport through it. There is need for a drug delivery system that embraces these concepts.

EP 798 305 A1 discloses cosmetic formulations comprising highly purified tocopheryl phosphate with a low amount of the bistocopheryl diphosphate having improved stability at neutral pH. The compositions are formulated with amphoteric or cationic surfactants. US 5,387,579 and JP 9 309 813 A disclose dermatologic compositions comprising tocopherol phosphates formulated with lecithin as emulsifier.

### Tocopherol

Vitamin E (tocopherol) is an essential part of skin dynamics and is known to be very important for skin health, with deficiency manifesting as a cornified, scaly delicate skin, thickened epidermis, scaling, lesions, chronic infection, inflammation and erythema. Vitamin E is the main naturally occurring lipid soluble agent protecting the skin from stress, and is the main lipid soluble agent protecting the cell membrane lipids from peroxidation.

Skin is subject to constant stress due to exposure to everyday elements - sun, wind and water. As a result, it is common for many cosmetic products such as lotions, moisturizers, shampoo and conditioners to contain vitamin E to assist in maintaining skin health and/or mitigate and/or prevent hair and skin damage resulting from ultraviolet radiation and other environmentally produced free radicals. In order to assist in maintaining skin health, it is necessary for the vitamin E to reach the target area of the dermis. The most direct method of achieving this targeting is to apply a topical formulation to the affected area. However, topical application of vitamin E to the skin using current formulations has variable success due to the skin's ability to erect an impenetrable barrier to many outside elements. It is critical to provide for the penetration of vitamin E through the epidermis to the dermis.

It is believed that topical formulations using tocopherol acetate have not been able to deliver adequate tocopherol beyond the epidermal layers, and therefore provide little benefit. Since tocopheryl acetate is a lipidic material requiring formulation with an oil-in-water emulsion, absorption from such a formulation is less than optimal.

The more bioactive salts of tocopheryl phosphate are beginning to also be used by cosmetic formulators. The product produced by known phosphorylation processes is a. mixture of mono-tocopheryl phosphate (TP), di-tocopheryl phosphate (T2P), mono-tocopheryl di-phosphate (TP2) and di-tocopheryl pyrophosphate (T2P2). TP is the desired product of known phosphorylation processes as it is hydrophilic. Some unreacted tocopherol (T) is also formed when T2P, TP2 and T2P2 are hydrolyzed to produce more of the desired hydrophilic component TP.

Before, the mixture may be used in cosmetic applications, the water solubility must be increased. T2P has poor water solubility and is therefore removed or modified in the prior art. This is time consuming, costly and, unless a proper solvent is chosen, can result in undesirable solvent residues.

### Formulation properties

Cosmetic products must also be aesthetic and pleasant to use. Of course, the products must be compatible with eye, skin and oral mucosa and have an overall toxicity profile appropriate for topical application: Applications which are designed for the oral mucosa and/or lip care must also be of an acceptable taste. If tocopheryl phosphates are to be used as a source of Vitamin E in foaming and cleansing products, then the hydrophobic substances need to be removed or modified to mitigate their foam suppression properties. Consumers have started to prefer transparent creams, lotions and gel vehicles for use on skin and hair, particularly for infant care, as this is a symbol of purity and mildness. Current tocopheryl phosphates cannot be used in such transparent products because they have limited water solubility and form opaque emulsions.

Finally, the opaque creams and lotions made with current tocopheryl phosphate mixtures have considerable stability problems at elevated temperatures and temperatures below freezing because of the limited water solubility of the tocopheryl phosphates.

There is thus a need for a drug delivery system which provides improved bioavailability and/or improved formulation properties.

### Summary of the invention

In this specification, the term "hydroxylated active" refers to tocopherol which has hydroxy groups which may be phosphorylated and (in the non-phosphorylated form) has a desired activity.

The hydroxylated active may be administered through oral, topical, inhalation, opthalmic, intravenous, enteral, parenteral or other appropriate presentations including those commercially utilized. The present invention relates to the discovery that the reaction product of one or more phosphate derivatives of tocopherol and a specific complexing agent has useful properties.

According to the invention there is provided a composition comprising the reaction product of:
(a) one or more phosphate derivatives of tocopherol and
(b) one or more complexing agents selected from the group consisting of arginine and substituted amines of the formula:

   NR¹R²R³

   wherein R¹ is selected from the group consisting of R⁴ and R⁴CO, wherein R⁴ is straight or branched chain mixed alkyl radicals from C6 to C22;
   R² and R³ are either both R⁵ or one R⁵ and one H, wherein R⁵ is chosen from the group consisting of -CH₂C(O)OX, -CH₂CH(OH)CH₂SO₃X, -CH₂CH(OH)CH₂OPO₃X, -CH₂CH₂C(O)OX, -CH₂CH₂CH(OH)CH2SO₃X or -CH₂CH₂CH(OH)CH₂OPO₃X and X is H, Na, K or alkanolamine; or
   wherein when R¹ is R⁴CO then R² is (CH₃) and R³ is -(CH₂CH₂)N(C₂H₄OH)-CH₂CH₂OPO₃Na or R² and R³ both are -(CH₂CH₂)N(C₂H₄OH)CH₂COOH.

Preferably, the mole ratio of phosphate derivatives of tocopherol to complexing agents is in the range of from 1:10 to 10:1. Preferably, the mole ratio of phosphate derivatives of tocopherol to complexing agents is in the range of from 1:2 to 2:1. A person skilled in the art will understand that the resultant composition will be a mixture of complexed and non-complexed phosphate derivatives of tocopherol depending on the amount of complexing agent used.

In a preferred embodiment there is provided a therapeutic formulation comprising (i) the reaction product of (a) and (b); and (ii) an acceptable carrier.

According to a second aspect of the invention, there is provided a method for improving the bioavailability of tocopherol comprising the step of reacting:
(a) one or more phosphate derivatives of tocopherol with < >
(b) one or more complexing agents as defined above

Preferably, there is a further step of adding an acceptable carrier.

There is also provided the use of the composition according to the invention as a cosmetic product.

The complexing agents increase the hydrophilic region on the hydroxylated active tocopherol to one that is of relatively high electronic charge and attractive to water molecules (more water soluble) which may cause the resulting complexes to be more bioavailable than the parent hydroxylated active. This is possibly due to delivery of a complex in the proximity of the intestinal wall in a derivative form which may result in efficient transport and higher tissue penetration. Further, the new complexes are weakly dissociated by water back to the original components of the complex thus releasing the drug, and the process does not require enzyme action or any other reaction to release the hydroxylated active. Complexation acts to convert lipids to surfactants allowing better emulsification of the active compound. There are a number of situations where complexation may be of value in the drug industry. Complexation may allow conversion of some injectable only formulations to orally available products by improving solubility. Complexation may also decrease injection time, increase predictability of bioavailability and allow further development of compounds whose low bioavailability has previously restricted clinical use.

It has been found that complexes of tocopheryl phosphates can be formed which are more soluble in water than the parent tocopheryl phosphates. Further, it is not necessary to remove any T2P prior to forming these complexes. As these complexes of tocopheryl phosphate are more hydrophilic, they are useful for cosmetic formulations. Phosphorylated tocopherol complexed with a tertiary amine acts as both a surfactant and active source of vitamin E, achieving higher bioavailability by quickly reaching the rate limiting CMC because of its higher water solubility or ability to form better emulsions and eventually chylomicrons if used in an oral or injectable formulation.

### DETAILED DESCRIPTION

The following terms are used throughout the specification and are intended to have the following meanings:

The term "electron transfer agent" is used herein to refer to the class of hydroxylated actives which (in the non-phosphorylated form) can accept an electron to generate a relatively stable molecular radical or accept two electrons to allow the compound to participate in a reversible redox system. The electron transfer agent that may be posphorylated is tocopherol including alpha-, beta-, and gamma-tocopherol.

The term "effective amount" is used herein to refer to an amount that reaches the target site in the human or animal in an amount that is measurably effective in the reduction of one or more symptoms.

The term "acceptable carrier" is used herein to refer to a carrier considered by those skilled in the drug, food or cosmetic arts to be non-toxic when used to treat humans, animals or plant in parenteral or enteral formulations. For example, ingestible compositions may include phospholipids such as lecithin, cephalins and related compounds.

The "phosphate derivatives of hydroxylated actives" comprises compounds covalently bound by means of an oxygen to the phosphorus atom of a phosphate group. The oxygen atom is typically derived from a hydroxyl group on the electron transfer agents (i.e. tocopherol). The phosphate derivative may exist in the form of a free phosphate acid, a salt thereof, a di-phosphate ester thereby including two molecules of electron transfer agent, a mixed ester including two different compounds selected from electron transfer agents, a phosphatidyl compound wherein the free phosphate oxygen forms a bond with an alkyl or substituted alkyl group. For example, tocopheryl phosphate may be provided mixed with ascorbyl phosphate or as an ascorbyl/tocopheryl phosphate. Similarly, tocopheryl phosphate could be combined with retinyl phosphates and/or ascorbyl phosphates. Phosphorylation may be accomplished by any suitable method. Preferably, the hydroxyl group in the hydroxylated active tocopherol, is phosphorylated using P₄O₁₀ according to the method disclosed in international patent application no. PCT/AU00/00452. Excess diphosphate derivatives may be hydrolyzed using methods known to those skilled in the art

Complexing agents are defined above A preferred complexing agent is N-lauryl imino di-propionate. Arginine is used when the composition needs to be ingestible.

The substituted amine complexing agents are amphoteric surfactants. The amphoteric surfactants may be ampholytic surfactants, that is, they exhibit a pronounced isoelectric point within a specific pH range; or zwitterionic surfactants, that is, they are cationic over the entire pH range and do not usually exhibit a pronounced isoelectric point. The substituted amines are those according to the following formula:

NR¹R²R³

wherein R¹ is chosen from the group comprising R⁴ or R⁴CO wherein R⁴ is straight or branched chain mixed alkyl radicals from C6 to C22.
R² and R³ are either both R⁵ or one R⁵ and one H wherein R⁵ is chosen from the group comprising -CH₂O(O)OX,-CH₂CH(OH)CH₂SO₃X,-CH₂CH(OH)CH2OPO₃X,-CH₂CH₂CO(O)X, CH₂CH₂CH(OH)CH₂SO₃X or-CH₂CH₂CH(OH)CH₂OPO₃X and X is H, Na, K or alkanolamine.

In addition, when R¹ is R⁴CO then R² may be (CH₃) and R³ may be -(CH₂CH₂)N(C₂H₄OH)-CH₂CH₂OPO₃Na or R² and R³ together may be -N(CH₂)₂N(C₂H₄OH)CH₂COOH.

Commercial examples are DERIPHAT sold by Henkel/Cognis, DEHYTON sold by Henkel/Cognis, TEGOBETAINE sold by Goldschmidt and MIRANOL sold by Rhone Poulenc.

These complexes may be formed by the reaction of one or more phosphate derivatives of one or more hydroxylated actives and the one or more complexing agents. Complexes of phosphate derivatives of hydroxylated actives can be made by neutralization of the free phosphoric acid ester directly during manufacture as a raw material suitable for compounding or in situ blending of the fixed sodium salts with the complexing agents during the finished cosmetic formulation process.

Formulations according to the present invention may contain from about 0.5 to about 30 weight percent tocopheryl phosphate derivative complexes, preferably from about 1 to about 20 wt percent, more preferably about - 2 to about 15 wt percent, and most preferably about 3 to about 12 wt percent, based on the total weight of the composition. A most preferred amount of tocopheryl phosphate derivative complexes is about 5 to about 10 wt. %.

The tocopheryl phosphate product produced by know phosphorylation processes is a mixture of mono-tocopheryl phosphates (TP); di-tocopheryl phosphate (T2P), mono-tocopheryl di-phosphate (TP2) and di-tocopheryl pyrophosphate (T2P2). The preferred result is usually a mixture of about 70/30 TP to T2P, however this results in limited water solubility. Before the mixture may be used in cosmetic application, the water solubility must be increased by forming complexes according to the invention.

Consumers have started to prefer transparent creams, lotions and gel vehicles for use on skin and hair, particularly for infant care, as this is a symbol of purity and mildness. Tocopheryl phosphates available prior to the present developments could not be used in such transparent products because they have limited water solubility and form opaque emulsions. Finally, the opaque creams and lotions made with such prior tocopheryl phosphate mixtures have considerable stability problems at elevated temperatures and temperatures below freezing because of the limited water solubility of the tocopheryl phosphates.

The tocopheryl phosphate derivative complexes are water-soluble and thus enhance the incorporation of tocopherol into water-based drug and cosmetic formulations. The water solubility of the complexes also increases the stability of the formulations over a wide range of temperatures and permits that manufacture of clear or transparent solutions. It has also been found that the complexes have increased surface activity and exhibit good foaming properties. This makes the complexes useful for cosmetic products such as cleansing agents and shampoo. The complexes provide stable cosmetic products, which are consumer acceptable while minimizing the problems with current tocopherol formulations.

Tocopheryl phosphate derivative complexes may be used in various products including antiperspirant sticks, deodorant sticks, sunscreens, facial cleansers, make-up removers, hair pomades, facial gels, oil-in- water moisturizers, lotions, conditioners, shampoos, conditioning shampoos, toothpaste, and foaming body washes.

The formulation of the invention may also be delivered in any suitable drug delivery system applied to the dermis including patches, gels, depots, plasters, aerosols and other sustained or delayed release systems designed to alter absorption kinetics.

A person skilled in the art will know what components may be used as the acceptable carrier for the compositions of the present invention. These will include excipients such as solvents, surfactants, emollients, preservatives, colorants, fragrances and the like.

There is also provided a method for increasing the water solubility and/or detergent properties of tocopheryl phosphate derivatives comprising the step of reacting phosphorylated tocopherol with one or more complexing agents as defined above.

### Examples

The invention will now be further explained and illustrated by reference to the following non-limiting examples.

The following components were used in the examples.

| | |
|---|---|
| Brij 72 | POE 2 Stearyl Ether ex Unichema Americas |
| Brij 721 | POE 21 Stearyl Ether ex ICI or Uniqema Americas |
| Carbopol 934 25% | Ex BF Goodrich |
| Cetiol LC | Ex Henkel/Cognis |
| Cetiol V | Ex Henkel/Cognis |
| Cetiol 3600 | Ex Henkel/Cognis |
| Citric acid | Ex Henkel/Cognis |
| Cocamide mea | Ex Croda |
| Cocamidopropylbetaine | 35% commercial formulation called Velvetex BA 35 ex |
| Dehyquart F | cationic conditioner ex Henkel/Cognis |
| Deriphat 160 | a 97% free flowing powder of lauryl-imino-dipionate ex |
| | Henkel/Cognis |
| Di-sodium-N-lauryl beta | Ex Henkel/Cognis |
| imino dipropionate | |
| Drakeol 9 | LT Mineral Oil ex Penreco |
| Emerest 132 | Stearic Acid ex Cognis |
| Emerest 2400 | Ex Henkel/Cognis |
| Emerest 2314 | Ex Henkel/Cognis. |
| Emulgin B2 | Ex Henkel/Cognis |
| Germaben II | Preservative ex Sutton Labs |
| Glycerin | Ex Henkel/Cognis |
| Isostearyl imidazoline | Miranol BM ex Rhone Poulenc |
| Kathon CG | Ex Rohm & Haas |
| Lanette O | Ex Henkel/Cognis |
| Lauramide mea | 100% commercial formulation called Standamide mea ex |
| | Henkel/Cognis |
| Microfine TiO₂ | Ex Tayca Corp |
| Mixed waxes | Carnube, paraffin, beeswax ex Croda |
| Natrasol 250 HHR | Ex Hercules |
| Oils emollients | Ex Croda |
| Pelemol PDD | Propylene Glycol Dicaprylate/ Dicaprate ex Phoenix |
| Peppermint Oil | Ex Firmenich |
| P₄O₁₀ | Ex China |
| Red iron oxide | Ex Warner Jenkinson |
| Silicones | polydimethylsiloxane polymers ex Dow Coming |
| Sodium lauryl-2-ether | 50% commercial formulation called Standapol ES 250 ex |
| sulfate | Henkel/Cognis |
| Sodium lauryl-3-ether | Ex Henkel/Cognis |
| sulfate | |
| Stearyl alcohol | Ex Croda |
| Tocopherol | Ex Hoffmann La Roche |
| Triethanolamine | Ex Henkel/Cognis |

### Example 1

Complexes of tocopheryl phosphates with ampholytic surfactant are prepared (Complex A).

Tocopherol was treated with P₄O₁₀ as outlined in PCT/AU00/00452 followed by hydrolysis of T₂P₂. The resultant tocopheryl phosphate mixture was reacted with an equimolar amount of di-sodium-N-lauryl beta imino dipropionate. The water content was adjusted to form a viscous slurry of about 30-70 % wt/wt total solids. The pH was adjusted X to 6.0-6.5 using either citric acid or additional beta imino surfactant. The slurry can be dried to the desired active concentration as a slurry or as a powder via any conventional drying process, i.e., oven-tray-drier and ground via a fitzmill to the desired particle size. The finished product was a free flowing white to off white powder or aqueous slurry, either of which was dispersible in water.

### Example 2

Complexes of tocopheryl phosphates with a zwitter-ionic surfactant were prepared from sodium salts of tocopheryl phosphates (Complex B). The sodium salts of Tocopheryl phosphate, the zwitterionic surfactant and Complex B were tested for foaming properties using the hand lather test.

### Part A: preparation of sodium salts of tocopheryl phosphates

The tocopherol was treated with P₄O₁₀ as outlined in PCT/AU00/00452 followed by hydrolysis of T₂P₂. After hydrolysis, the tocopheryl phosphates were neutralized to the mono- and di-sodium salts. The resulting product was a viscous tan paste with a Gardiner color of about 8-10 and a pH of 8.0-8.5.

A 2% wt/wt aqueous solution of this paste formed an emulsion with a particle size of at least 10 microns (milky), which produced little or no foam as per hand lathering tests. The emulsion was unstable after two days at 50°C and after one week at ambient room temperature.

### Part B: Preparation of Complex B

Forty parts of the tocopheryl phosphates paste formed in part A were mixed with 60 parts of cocamidopropylbetaine containing sufficient water to form a 40% wt/wt slurry using a Waring blender. The weight ratio of betaine to tocopheryl phosphate was 1.5:1. The pH was adjusted to 6.0-6.5 using citric acid.

A 5% active solution containing 40% tocopheryl phosphate (equivalent to the 2% wt/wt solution prepared in part A) formed a translucent emulsion with particles of less than 2 microns, which produced copious foam via hand lathering tests. This foam was denser than the foam produced by either the cocamidopropylbetaine or the tocopheryl phosphates from part A alone. The hand lathering tests showed that a residual amount of the product provided a tactile skin feel - an indication of adherence to skin and keratin fiber.

### Properties

| | |
|---|---|
| Appearance | a translucent emulsion |
| pH as is | 6.0-6.5 |
| Lather | Copious foam |
| Stability 50°C | Stable and clear at least one week |

### Example 3

In this example, complexes were dry blended. Certain complexes can also be dry blinded prior to either forming slurry or compounding in situ.

Forty parts of mixed sodium salts of tocopheryl phosphates were ground to a powder via freeze drying and mixed in a Warming blender with sixty parts of Deriphat 160 (a 97% free flowing powder) for twenty minutes to form a homogeneous free flowing powder consisting of di-sodium lauryl-imino-dipropionate tocopheryl phosphate complexes.

### Example 4

In this example, a hand and body wash was formulated using Complex A from Example 1.

The tocopheryl phosphate salts were heated with water until clear and homogeneous. Ammonium lauryl sulfate was added and mixed until clear. Cocamide Mea was added and the pH was adjusted to 5.5 to 6.0 with citric acid. The solution was cooled to 35°C and Kathon CG was added and mixed for ten minutes. Deionized water was added to complete the finished product to 100 parts total. Sodium chloride was added to adjust the viscosity to 4000-5000 centipoises at 25°C.

| Ingredient | %wt/wt |
|---|---|
| Complex A from Example 1 | 10 |
| *Ammonium* lauryl sulfate 30% | 40 |
| Cocamide mea | 2 |
| Kathon cg | 0.05 |
| NaCl, citric acid, deionized water | qs to 100% |

| Properties | |
|---|---|
| Viscosity at 25°C | 4000-5000 |
| pH as is | 5.5 to 6.0 |

### Example 5

A foaming shower gel for skin/hair for sports and chlorine scavenging was formulated using Complex B from Example 2.

Fifteen parts of the 40% Complex B from Example 2 were mixed with fifty parts of water and heated to 50°C and mixed until clear and homogeneous. Thirty parts of 30% active sodium lauryl-3-ether sulfate were added and mixed until the solution was clear and homogeneous. Three parts of cocamide mea were added and the pH was adjusted to 6-6.5 with lactic acid followed by cooling to 3.5C. 0.1 parts of preservative kathon cg 0.2 were added followed by deionized water to 100% total to give the following formula:

| Ingredient % wt/wt | |
|---|---|
| Complex B from Example 2 (40%) | 15 |
| Sodium lauryl-3-ether sulfate | 35 |
| Cocamide mea | 3 |
| Preservative, color, fragrance, deionized water | Qs to 100% |

| Properties | |
|---|---|
| Viscosity | 25,000 cps |
| pH as is @ 25°C | 6.0-6.5 |

The complex can also be made in situ while compounding the finished cosmetic.

### Example 6

A sports shampoo and shower gel was prepared with in situ formation of the tocopheryl phosphate complexes.

Sixty parts of deionized water were heated to 60-70°C followed by the addition of seven parts of 35% cocamidopropylbetaine and mixed until clear. Two parts of mixed sodium salts of tocopheryl phosphate were added and mixed until clear and homogeneous. Twenty-five parts of 50% sodium lauryl-2-ether sulfate were added and mixed until the solution was clear. Three parts of cocamide mea were, added and mixed until clear. The pH was adjusted to 5.0-5.5 with citric acid and cooled to 35°C. The preservative, color and fragrance were added and the batch was adjusted to 100 % with deionized water to provide the following formula.

| Ingredient | % wt/wt |
|---|---|
| Sodium lauryl-2-ether sulfate | 25 |
| Cocamidopropylbetaine | 7 |
| Sodium tocopheryl phosphates | 2 |
| Lauramide mea | 3 |
| Citric acid | Qs |
| Preservative and deionized water | Qs to 100% |

| Properties | |
|---|---|
| Appearance | clear viscous gel |
| viscosity | 25,000 cps |
| pH as is | 5.0-6.0 |
| Lather | rich lubricious |
| Stability 50°C | Stable and clear for 2 weeks |
| Freeze/Thaw: 2 cycles | Stable |

The gels of this type often require a rheology modification using semi-synthetic polymers such as cellulosic gums as needed.

### Example 7

An economy conditioning shampoo was prepared from the formulation in Example 6.

The product from Example 6 was diluted with deionized water at a wt/wt ratio of 75 parts of Example 6 to twenty five parts of water to provide a shampoo with a viscosity of 3000 cps at 25°C. the product was clear and stable as per Example 6. The product is high foaming/cleansing with the additional benefit of providing perceived body or fullness to hair.

Applications of the complex salts designed for non-foaming areas such as hair conditioners, body and facial creams, sun, shave and lip products etc, can be produced via using a higher alkyl chain as the hydrophobic group on the amphoteric portion of the complex and/or the use of cationic salts such as those used in hair conditioners. These products can be made using any of the above methods of complex formation.

### Example 8 (Reference)

A rinse-off hair conditioner was prepared using tocopheryl phosphates with a cationic surfactant to form a complex.

| Ingredient | % wt/wt |
|---|---|
| Dehyquart F | 2 |
| Tocopheryl phosphates | 2 |
| Stearyl alcohol | 1 |
| Brij 721 2 | |
| Natrasol 250 HHR | 1 |
| Citric acid | 0.5 |
| Preservative, dye and deionized water | Qs to 100% |

| Properties | |
|---|---|
| Appearance | clear viscous gel |
| Viscosity | 5000 cps |
| pH as is | 4-5 |
| Lather | rich lubricious |
| Stability 50°C | Stable for 2 weeks |
| Freeze/Thaw - 2. cycles | Stable |

### Example 9 (Reference)

A facial anti aging crème was prepared using an isostearyl analogue of imidazoline (amphoteric surfactant).

| **Ingredient** | **% wt/wt** |
|---|---|
| Part A | |
| Isostearyl imidazoline | 1.0 |
| Emulgin B2 | 1.4 |
| Emerest 2400 | 2.0 |
| Lanette O | 2.0 |
| Emerest 2314 5.0 | |
| Cetiol LC | 3.5 |
| Cetiol V | 3.5 |
| Cetiol 3600 | 3.0 |

| Part B | |
|---|---|
| Carbopol 934 (25%) | 10.0 |
| Tocopheryl phosphate | 2.0 |
| Deionized water | 57.6 |
| Glycerin | 5.0 |

| Part C | |
|---|---|
| Triethanolamine | 0.5 |

| Part D | |
|---|---|
| Germaben II preservative | 1.0 |

Mix parts A and B in separate vessels and heat to 80°C. Add A to B and mix at 80°C for 10 minutes. Cool to 60°C then add C. Cool to 60°C then add D.

| Properties | |
|---|---|
| Appearance | stable white crème with pleasant tactile skin feel |
| Stability 50°C | Stable for 1 month |
| Freeze/Thaw-2cycles | Stable |

### Example 10 (Reference)

A lanolin free lipstick was prepared using the complex in Example 9.

| **Ingredient** | **% wt/wt** |
|---|---|
| Isostearyl imidazolinium tocopheryl phosphate | 3 |
| Mixed waxes | 30 |
| Oils emollients | 45 |
| Red iron oxide | 5 |
| Microfine TiO₂ | 5 |
| Silicones | as to 100% |

Stable lipstick with good pay-off and pleasant taste.

### Example 14

A lotion was prepared as follows. The following ingredients are mixed.

| **Ingredient** | **w/w percent** |
|---|---|
| cetyl alcohol | 0.75 |
| C12-15 alcohol benzoate | 5 |
| butylated hydroxyanisole | 0.1 |
| PEG-100 stearate | 0.25 |
| water, deionized or distilled | 70.4 |
| propylene glycol | 3.0 |
| tocopheryl phosphate complex (TPC of Ex. 2) | 10.5 |
| acetone | 10.0 |

### Example 15

A cream was manufactured by mixing the following ingredients:

| **Ingredients** | **w/w percent** |
|---|---|
| cetyl-stearyl alcohol | 1.25 |
| C12-15 alcohol benzoate | 5 |
| butylated hydroxyanisole | 0.01 |
| PEG-100 stearate | 0.85 |
| water, deionized or distilled | 69.1 |
| propylene glycol | 3 |
| tocopheryl phosphate complex (TPC of Ex 1) | 10.5 |
| acetone | 10 |

### Example 14

A gel according to the present invention was prepared by combining the following ingredients.

| **Ingredient** | **w/w percent** |
|---|---|
| water, deionized or distilled | 50.65 |
| Veegum^{®} (R.T.Vanderbilt Co.) | 1.5 |
| carboxy vinyl polymer (acid) | 1 |
| diisopropanolamine | 0.75 |
| ethyl alcohol, 200° | 30.1 |
| tocopheryl phosphate complex (TPC of Ex.1) | 15 |

### Example 15

Fifteen mg of Carbomer (15 mg) was added to distilled water (495 mg) while stirring. Stirring was continued for about 45 minutes. A solution of sodium hydroxide (4.09 mg) in distilled water (4.9 ml) was added and stirring as continued for 10 minutes. Ethyl alcohol (150 ml) and methyl salicylate (1 mg) were added to the stirred solution, followed by tocopheryl phosphate complex (50% TP complex of Example 1-50% water) (400 mg), and distilled water (80 ml). The resulting mixture was stirred until a smooth gel was obtained.

### Example 16

The following gel formulation was prepared according to the procedure described in Example 15.

| **Ingredient** | **w/w percent** |
|---|---|
| tocopheryl phosphate complex | 20 |
| tetracycline | 2 |
| ethylalcohol | 20 |
| PEG-8 caprate | 6 |
| colloidal mg aluminum silicate | 2.5 |
| hydroxyethylmethyl cellulose | 0.75 |
| citric acid | 0.05 |
| water | Q.S. |

### Example 17

Aqueous gel compositions were prepared according to the following formulation:

| **Ingredient** | **w/w percent** |
|---|---|
| tocopheryl phosphate complex | 15 |
| retin A | 0.5 |
| carbomer^{®}940 | 1 |
| sodium hydroxide to desired pH | |
| water | QS |

### Example 18

A lotion with sunscreen was prepared as follows.

| | **Ingredients** | **%w/w** |
|---|---|---|
| A | Brij 72 (POE 2 Stearyl Ether) | 0.5 |
| | Emerest 132 (Stearic Acid) | 2.0 |
| | Pelemol PDD (Propylene Glycol Dicaprylate/ Dicaprate) | 10.0 |
| | Drakeol 9 (LT Mineral Oil) | 9.0 |
| | Brij 721 (POE 21 Stearyl Ether) | 1.0 |
| | Octylmethoxy Cinnamate | 7.0 |
| | Benzophenane-3 | 2.0 |
| | Dicoming 200 Fluid (Dimethicane) | 1.0 |
| | Propyl Paraben | 0.1 |
| B | Cabopol Ultrez 10 Slurry 3% | 5.0 |
| | Water | 10.0 |
| C | TEA 99% | 1.2 |
| | Water Distilled | 10.0 |
| | Methyl Paraben | 0.25 |
| | Lauryl Imino Dipropionic Acid Tocopheryl Phosphate - 40% with DMDMH | 7.5 |
| | Water Distilled q.s. | 33.45 |

Heat A and C separately to 80°C. Add A to C while mixing with an homogenizer for 2 to 3 min. Remove the mixture from the homogenizer, add B (which has been heated to 70°C) and then cool to room temperature.

### Example 19

A toothpaste was prepared as follows:

| | **Ingredients** | **%w/w** |
|---|---|---|
| A | Sorbitol USP | 15.0 |
| | 40% Lauryl Imino Dipropionic Acid Tocopheryl Phosphate | 7.5 |
| B | Glycerin USP 96% | 10.0 |
| | Triclosan | 0.3 |
| | Na-Saccharin USP 40/60 Mesh | 0.2 |
| | Veegum D-Granular | 2.0 |
| | Peppermint Oil | 1.1 |
| | Stepanol WA/100 (Na-Lauryl Sulfate) | 2.2 |
| C | Veegum HF-6% (Ag/Al Silicate) | 16.64 |
| | Blue #1 FD+C (0.6%) | 0.06 |
| D | Na-CMC 7 H 5% | 45.0 |

Mix together the components of A, then add all items of B to A and mix until uniform. Add C and mix until uniform. Finally, add D slowly mixing until uniform.

### Example 20

A tocopheryl phosphate amphoteric complex formulations is prepared as follows:

| **Ingredient** | **% w/w** |
|---|---|
| di-sodium alpha tocopheryl phosphate N-lauryl imino dipropionate complex | 30% |
| water | 67% |
| lanolin creme | 3% |

### Example 21

Di-sodium alpha tocopheryl phosphate N-lauryl imino dipropionate complex (a 60/40-N-lauryl imino dipropionate / mixed-phosphate weight ratio) was analyzed in tests as follows.

### ³¹P NMR

³¹P spectra were carried out at ambient temperature using a Bruker DPX300 spectometer.

The complex mixture was dissolved in CDCl₃. The spectrum had a single peak at -2.9 ppm and a single peak at -7.9 ppm. There was also a small peak for inorganic phosphates at 1.0 ppm.

The spectrum for pure di-sodium mono-tocopheryl phosphate (dissolved in THF/H₂O (2:1)) consisted of a single peak at 1.1 ppm. The spectrum for pure sodium di-tocopheryl phosphate (dissolved in THF/H20 (2:1)) consisted of a single peak at -7.5.

From this information it can be concluded that a mono-tocopheryl phosphate N-lauryl imino dipropionate complex formed and corresponds to the peak at -2.9 ppm.

### Electrospray mass spectrometry

The complex product was then analysed by electrospray mass spectrometry on a Micromass Platform II spectrometer using an accelerating voltage of 40V. The spectrum had peaks at 328 for N-lauryl imino dipropionate, 509 for mono-tocopheryl phosphate, 838 for mono-toropheryl phosphate N-lauryl imino dipropionate complex and 922 for di-tocopheryl phosphate.

The mono-tocopheryl phosphate N-lauryl imino dipropionate complex survived the intense accelerating field. A typical salt would dissociate in such an electron field. Therefore it is apparent that mono-tocopheryl phosphate N-lauryl imino dipropionate complex is not a typical salt.

### Osmometry

A vapour pressure osmometer was used to investigate the dissociation of the di-sodium alpha tocopheryl phosphate N-lauryl imino dipropionate complex by comparing the lowering of the equilibrium temperature to give an identical partial pressure of water vapour ground a drop of pure water versus various solutions as an indication of the relative moles of solute. The instrument does not output absolute temperature but instead gives an arbitrary scale that is directly related to sodium chloride as a solute, thus for 0.1M sodium chloride the output was a 29 unit effect.

N-lauryl imino dipropionate alone gives three ions and at 0.05M the effect was 38 units. If the complex was readily dissociated, then the additional tocopheryl phosphate would be expected to increase the effect in the ratio 3:5 by the addition of the charged amino group as a cation and tocopheryl hydrogen phosphate anion. However, addition of 0.05M of tocopheryl phosphate to the 0.05M N-lauryl imino dipropionate resulted in a solution with 36 units.

This result remonstrates that the complex is not ionised in water. Therefore the complex was not a typical salt where the ionic bonds are readily broken by high dielectric solvents such as water. The behaviour of the complex resembles potassium ferricyanide where the ferricyanide ion is not deemed to be a salt because the iron-cyanide bond is not broken by water as a solvent, such ions are called complexes.

### Example 21

Di-sodium tocopheryl phosphate (1.3 g) was dissolved in 2 ml of water. Arginine hydrochloride (0.5 g) was added and the mixture was intimately mixed for one hour. The mixture increased in viscosity until a gel was formed indicating that a reaction had occurred.

The complex product was then analysed by electrospray mass spectrometry on a Micromass Platform II spectrometer using an accelerating voltage of 40V. The spectrum showed peaks at 510 (tocopheryl phosphate) and 683 (tocopheryl phosphate arginine complex) mass units. The 683 peak indicates the bond between arginine and tocopheryl phosphate survived the intense accelerating field and thus is very strong. A typical salt would not have survived such a field.

### Example 22 (Reference)

Amoxycillin was treated with P₄O₁₀ as outlined in PCT/AU00/00452 to prepare its phosphate derivatives. 445.4 g (1 mole) of amoxycillin phosphoric acid was dispersed in 2 L of water and 327.6 g of Deriphat was added and mixed for 10 minutes to generate the complex. The solution was then dried to give the complex. The complex was shown to be readily soluble in water.

### Example 23 (Reference)

Timolol eye drops are utilized to decrease aqueous secretion from the ciliary epithelium and alleviate symptoms of open-angle glaucoma. Sterile opthalmic drops containing 2.5 mg/ml of timolol can be mixed with 3 mg/ml hypromellose solution to reduce "stinging" sensation and improve product absorption.

When 30 mg of timolol is mixed with phosphoric acid and excess fatty acid in sterile water, timolol phosphate is formed. Deriphat was added in an amount equimolar to the timolol phosphate added and was mixed for 10 minutes to form a complex which is more water soluble than the timolol hypromellose solution.

### Example 24 (Reference)

Di-sodium ubiquinyl phosphate (0.3 g) was dissolved in 2 ml of water. Deriphat (0.14g) was dissolved in 2 ml water and then added to the ubiquinyl phosphate mixture and intimately mixed for one hours. The mixture increased in viscosity until a gel formed indicating that a reaction had occurred.

The product was analyzed by electrospray mass spectrometry on a Micromass Platform II spectrometer using an accelerating voltage of 40V. The spectrum showed peaks at 945 (ubiquinyl phosphate) and 1273 (ubiquinyl phosphate N-lauryl amino dipropionate complex). The 1273 peak indicates the bond between N-lauryl imino dipropionate and ubiquinyl phosphate survived the intense accelerating field and thus is very strong. A typical salt would not have survived such a field.

### Example 25

The skin penetration properties of complexed and non-complexed tocopheryl phosphate (non-complexed (sodium salts)) were compared relative to tocopheryl acetate.

### Test formulations

The test materials were made up on the basis of 5% mixed actives tocopherol (T), tocopheryl phosphate (TP) and tocopheryl diphosphate (T2P) or tocopheryl acetate in a vehicle consisting of 95/5 distilled water/ethanol with pH adjusted (if necessary to 6.5-7.0 with citric acid or dilute NaOH).

### Tocopheryl phosphate complexes (TPC)

The TPC used was lauryl-imino di-propionic acid tocopheryl phosphate; a surface-active amphoteric phosphate ester complex formed from lauryl imino propionic acid (Deriphat 160) and tocopheryl phosphates.

| Active | TPC |
|---|---|
| | (micrograms per applied dose) |
| tocopheryl phosphate | 188 |
| di-tocopheryl phosphate | 713 |
| Tocopherol | |

The solution for TPC was based on 40% active mixed phosphates as the latter was reacted/combined in a 60/40-amphoteric/mixed-phosphate weight ratio (1.9-1 mole ratio). 12.5 w/w % of TPC was dissolved in 87.5 w/w % of the 95/5 water/ethanol mixture.

### Di-sodium salt of mono- and di-tocopheryl phosphates (DSS)

DSS was similar in TP and T2P content, however, unlike TPC, DSS existed as the mixed sodium salts. A slurry of 6.25 w/w % of 80% DSS in 93.75 w/w % of the 95/5-water/ethanol mixture was prepared.

| Active | DSS |
|---|---|
| | (micrograms per applied dose) |
| tocopheryl phosphate | 252 |
| di-tocopheryl phosphate | 1194 |
| tocopherol | 24 |

### Tocopheryl Acetate (TA)

Tocopheryl acetate was obtained from Roche/BASF. 5.0 w/w % of TA was dispersed in 95-0 w/w% of 95/5 water/ethanol mixture.

### Method

The test formulations were evaluated in *in vitro* human skin penetration studies. Samples were analyzed for the mono- and di-tocopheryl phosphates, free alpha-tocopherol, and tocopheryl acetate by high performance liquid chromatography (HPLC). The tests were conducted by DermTech International (San Diego, CA). Human cadaver skin was obtained and prepared. Each formulation was evaluated on triplicate sections from each donor at a topically applied dose of 5 µL/cm². Receptor solutions were collected over 48 hours at pre-selected time intervals. After 48 hours the skin surface was washed with isopropyl alcohol, and the skin was collected and split into epidermis and dermis. The skin sections were extracted with isopropyl alcohol. All collected samples were processed and assayed for tocopherol, tocopheryl acetate, tocopheryl phosphate and di-tocopheryl phosphate.

Mass balance from the samples is between 80-120% of the applied dose.

No tocopherols were observed in the receptor solution. This could be a result of amounts being below limits of detection, or degradation of the various tocopherol species into other, as yet uncharacterized, compounds.

**Table 1: Skin Penetration Study**

| Percent Distribution of Tocopherols Recovered across Samples wt/wt % | | | |
|---|---|---|---|
| **DSS** | **T** | **TP** | **T2P** |
| Surface Wash | 65.05 | 41.40 | 56.05 |
| Epidermis | 26.74 | 47.06 | 37.31 |
| Dermis | 8.24 | 11.42 | 6.62 |
| Dermis/Epidermis Ratio | 0.31 | 0.24 | 0.18 |
| | | | |

| **TPC** | **T** | **TP** | **T2P** |
|---|---|---|---|
| Surface Wash | 50.00 | 48.82 | 70.92 |
| Epidermis | 35.99 | 24.55 | 16.67 |
| Dermis | 14.07 | 26.62 | 12.36 |
| Dermis/Epidermis Ratio | 0.39 | 1.08 | 0.74 |
| | | | |

| **TA** | **Tocopheryl Acetate** | | |
|---|---|---|---|
| Surface Wash | 91.48 | | |
| Epidermis | 7.13 | | |
| Dermis | 1.39 | | |
| Dermis/Epidermis Ratio | 0.20 | | |

### Summary Of Results

(a) The T, TP and T2P in the DSS and TPC formulations penetrate into the skin more effectively than TA.
(b) TPC is a better delivery system than DSS as shown by a higher TP penetration ratio into the dermis/epidermis.
(c) The enhanced penetration of the tocopheryl phosphates from TPC, is most likely the result of the TPC surface-active properties. The TPC is more effective in lowering the surface tension at the liquid/skin interface compared to both DSS and TA. The latter is the most hydrophobic of the three test materials and forms a poor dispersion in the water/alcohol vehicle.

The word 'comprising' and forms of the word 'comprising' as used in this description and in the claims does not limit the invention claimed to exclude any variants or additions.

Modifications and improvements to the invention will be readily apparent to those skilled in the art. Such modifications and improvements are intended to be within the scope of this invention.

## Claims

1. A composition comprising the reaction product of:
(a) one or more phosphate derivatives of tocopherol; and
(b) one or more complexing agents selected from the group consisting of arginine and substituted amines of the formula:
NR¹R²R³
wherein R¹ is selected from the group consisting of R⁴ and R⁴CO, wherein R⁴ is straight or branched chain mixed alkyl radicals from C6 to C22;
R² and R³ are either both R⁵ or one R⁵ and one pH, wherein R⁵ is chosen from the group consisting of -CH₂C(O)OX, -CH₂CH(OH)CH₂SO₃X, -CH₂CH(OH)CH₂OPO₃X, -CH₂CH₂C(O)OX, -CH₂CH₂CH(OH)CH₂SO₃X or -CH₂CH₂CH(OH)CH₂OPO₃X and X is H, Na; K or alkanolamine; or
wherein when R¹ is R⁴CO then R² is (CH₃) and R³ is -(CH₂CH₂)N(C₂H₄OH)-CH₂CH₂OPO₃Na or R² and R³ - both are -(CH₂CH₂)N(C₂H₄OH)CH₂COOH.

2. The composition according to claim 1, wherein the complexing agent is arginine.

3. The composition according to claim 1, wherein the complexing agent is a substituted amine NR¹R²R³ as defined in claim 1.

4. The composition according to claim 1, wherein the complexing agent is N-lauryl imino di-propionate or disodium-N-lauryl beta imino dipropionate.

5. The composition according to any of the preceding claims, wherein the mole ratio of the phosphate derivatives of tocopherol to complexing agents is in the range of from 1:10 to 10:1, preferably in the range of from 1:2 to 2:1.

6. The composition according to any of the preceding claims, wherein the tocopherol is alpha-tocopherol, beta-tocopherol or gamma-tocopherol.

7. The composition according to any of the preceding claims, wherein the tocopherol is alpha-tocopherol.

8. The composition according to claim 1, wherein the phosphate derivative is a free phosphate acid, a salt thereof, a diphosphate ester, mixed ester, or a phosphatidyl compound wherein the free phosphate oxygen forms a bond with an alkyl or substituted alkyl group.

9. The composition according to claim 8, wherein the salt is a sodium salt of tocopheryl phosphate.

10. The composition according to claim 1, wherein there is more than one phosphate derivative of tocopherol.

11. The composition according to claim 1, wherein there is more than one phosphate derivative of more than tocopherol.

12. The composition according to claim 1, wherein the composition comprises from about 0.5 to about 30 weight percent, preferably from about 1 to about 20 wt percent, more preferably from about 2 to about 15 wt percent, and most preferably from about 3 to about 12 wt percent, of the reaction product based on the total weight of the composition.

13. The composition according to claim 12, wherein the composition comprises from about 5 to about 10 weight percent of the reaction product based on the total weight of the composition.

14. The composition according to any of the preceding claims further comprising a carrier.

15. Use of the composition as defined in any of claims 1 to 14 in a cosmetic product.

16. The use according to claim 15, wherein the cosmetic product is a cleansing agent, an antiperspirant stick, a deodorant stick, a sunscreen, a facial cleanser, a make-up remover, a hair pomade, a facial gel, an oil-in-water moisturizer, a lotion, a conditioner, a shampoo, a conditioning shampoo, a toothpaste or a foaming body wash.

17. A therapeutic formulations for use on humans, animals or plants comprising the composition according to any of claims 1 to 14 and an acceptable carrier.

18. A method for improving the bioavailability of tocopherol comprising the step of reacting:
(a) one or more phosphate derivatives of tocopherol; with
(b) one or more of the complexing agents defined in any of claims 1 to 4.

## Patentansprüche

1. Zusammensetzung, umfassend das Umsetzungsprodukt von:
(a) einem oder mehreren Phosphatderivaten von Tocopherol; und
(b) einem oder mehreren Komplexbildnern, ausgewählt aus Arginin und substituierten Aminen der Formel:
NR¹R²R³
wobei R¹ aus R⁴ und R⁴CO ausgewählt ist, wobei R⁴ geradkettige oder verzweigte gemischte C6- bis C22-Alkylreste darstellt;
R² und R³ entweder beide R⁵ sind, oder einer R⁵ und einer H ist, wobei R⁵ aus -CH₂C(O)OX, -CH₂CH(OH)CH₂SO₃X, -CH₂CH(OH)CH₂OPO₃X, -CH₂CH₂C(O)OX, -CH₂CH₂CH(OH)CH₂SO₃X oder -CH₂CH₂CH(OH)CH₂OPO₃X ausgewählt ist, wobei X die Bedeutung H, Na, K oder Alkanolamin hat; oder
wobei wenn R¹ die Bedeutung R⁴CO hat, R² die Bedeutung (CH₃) hat und R³ die Bedeutung -(CH₂CH₂)N(C₂H₄OH)-CH₂CH₂OPO₃Na hat oder R² und R³ beide -(CH₂CH₂)N(C₂H₄OH)CH₂COOH sind.

2. Zusammensetzung gemäß Anspruch 1, wobei der Komplexbildner Arginin ist.

3. Zusammensetzung gemäß Anspruch 1, wobei der Komplexbildner ein wie in Anspruch 1 definiertes substituiertes Amin NR¹R²R³ ist.

4. Zusammensetzung gemäß Anspruch 1, wobei der Komplexbildner N-Lauryliminodipropionat oder Dinatrium-N-lauryl-beta-iminodipropionat ist.

5. Zusammensetzung gemäß einem der vorhergehenden Ansprüche, wobei das Molverhältnis der Phosphatderivate von Tocopherol zu den Komplexbildnern im Bereich von 1:10 bis 10:1, vorzugsweise im Bereich von 1:2 bis 2:1 liegt.

6. Zusammensetzung gemäß einem der vorhergehenden Ansprüche, wobei das Tocopherol alpha-Tocopherol, beta-Tocopherol oder gamma-Tocopherol ist.

7. Zusammensetzung gemäß einem der vorhergehenden Ansprüche, wobei das Tocopherol alpha-Tocopherol ist.

8. Zusammensetzung gemäß Anspruch 1, wobei das Phosphatderivat eine freie Phosphorsäure, ein Salz davon, ein Diphosphatester, ein gemischter Ester, oder eine Phosphatidylverbindung ist, wobei der freie Phosphatsauerstoff eine Bindung mit einem Alkyl- oder substituierten Alkylrest bildet.

9. Zusammensetzung gemäß Anspruch 8, wobei das Salz ein Natriumsalz von Tocopherylphosphat ist.

10. Zusammensetzung gemäß Anspruch 1, wobei mehr als ein Phosphatderivat von Tocopherol vorhanden ist.

11. Zusammensetzung gemäß Anspruch 1, wobei mehr als ein Phosphatderivat von mehr als einem Tocopherol vorhanden ist.

12. Zusammensetzung gemäß Anspruch 1, wobei die Zusammensetzung von etwa 0,5 bis etwa 30 Gew.-%, vorzugsweise von etwa 1 bis etwa 20 Gew.-%, stärker bevorzugt von etwa 2 bis etwa 15 Gew.-%, und am meisten bevorzugt von etwa 3 bis etwa 12 Gew.-% des Umsetzungsproduktes, bezogen auf das Gesamtgewicht der Zusammensetzung, umfasst.

13. Zusammensetzung gemäß Anspruch 12, wobei die Zusammensetzung von etwa 5 bis etwa 10 Gew.-% des Umsetzungsproduktes, bezogen auf das Gesamtgewicht der Zusammensetzung, umfasst.

14. Zusammensetzung gemäß einem der vorhergehenden Ansprüche, die des Weiteren einen Träger umfasst.

15. Verwendung der wie in einem der Ansprüche 1 bis 14 definierten Zusammensetzung in einem Kosmetikprodukt.

16. Verwendung gemäß Anspruch 15, wobei das Kosmetikprodukt ein Reinigungsmittel, ein Antitranspirant-Stick, ein Deodorant-Stick, ein Sonnenschutzmittel, ein Gesichtsreinigungsmittel, ein Makeup-Entferner, eine Haarpomade, ein Gesichtsgel, ein Öl-in-Wasser-Feuchtigkeitsmittel, eine Lotion, ein Conditioner, ein Shampoo, ein Conditioner-Shampoo, eine Zahnpasta oder ein schäumendes Körperreinigungsmittel ist.

17. Therapeutische Formulierung zur Verwendung bei Menschen, Tieren oder Pflanzen, umfassend die Zusammensetzung gemäß einem der Ansprüche 1 bis 14 und einen verträglichen Träger.

18. Verfahren zur Verbesserung der Bioverfügbarkeit von Tocopherol, umfassend den Schritt des Umsetzens:
(a) eines oder mehrerer Phosphatderivate von Tocopherol; mit
(b) einem oder mehreren der in einem der Ansprüche 1 bis 4 definierten Komplexbildner.

## Revendications

1. Composition comprenant le produit de réaction de :
(a) un ou plusieurs dérivés de phosphate de tocophérol ; et
(b) un ou plusieurs agents complexants choisis dans le groupe constitué d'arginine et d'amines substituées de formule :
NR¹R²R³
dans laquelle R¹ est choisi dans le groupe constitué de R⁴ et R⁴CO, dans lequel R⁴ est un radical alkyle mélangé à chaîne droite ou ramifiée de C6 à C22 ;
R² et R³ sont soit tous les deux un R⁵ ou un R⁵ et un H, dans lesquels R⁵ est choisi dans le groupe constitué de -CH₂C(O)OX, -CH₂CH(OH)CH₂SO₃X, -CH₂CH(OH)CH₂OPO₃X, -CH₂CH₂C (O)OX, -CH₂CH₂CH(OH)CH₂SO₃X, ou -CH₂CH₂CH(OH)CH₂OPO₃X et X est un H, Na, K ou une alcanolamine ; ou
dans laquelle quand R¹ est un R⁴CO, alors R² est un (CH₃) et R³ est un -(CH₂CH₂)N(C₂H₄OH)-CH₂CH₂OPO₃Na ou R² et R³ sont tous deux un - (CH₂CH₂)N(C₂H₄OH)CH₂COOH.

2. Composition selon la revendication 1, dans laquelle l'agent complexant est l'arginine.

3. Composition selon la revendication 1, dans laquelle l'agent complexant est une amine substituée NR¹R²R³ comme définie dans la revendication 1.

4. Composition selon la revendication 1, dans laquelle l'agent complexant est le N-lauryl imino dipropionate ou le disodium-N-lauryl bêta imino dipropionate.

5. Composition selon l'une quelconque des revendications précédentes, dans laquelle le rapport en mole entre les dérivés de phosphate de tocophérol et les agents complexants est compris entre 1 :10 et 10 :1, de préférence entre 1 :2 et 2 :1.

6. Composition selon l'une quelconque des revendications précédentes, dans laquelle le tocophérol est l'alpha-tocophérol, le bêta-tocophérol ou le gamma-tocophérol.

7. Composition selon l'une quelconque des revendications précédentes dans laquelle le tocophérol est l'alpha-tocophérol.

8. Composition selon la revendication 1, dans laquelle le dérivé de phosphate est un acide de phosphate libre, son sel, un diphosphate ester, un ester mélangé, ou un composé de phosphatidyle dans lequel l'oxygène de phosphate libre forme une liaison avec un groupe alkyle ou alkyle substitué.

9. Composition selon la revendication 8, dans laquelle le sel est un sel de sodium de phosphate de tocophéryle.

10. Composition selon la revendication 1, dans laquelle il y a plus d'un dérivé de phosphate de tocophérol.

11. Composition selon la revendication 1, dans laquelle il y a plus d'un dérivé de phosphate de plusieurs éléments de tocophérol.

12. Composition selon la revendication 1, dans laquelle la composition comprend d'environ 0,5 à environ 30 pour cent en poids, de préférence d'environ 1 à environ 20 pour cent en poids, de manière davantage préférée d'environ 2 à environ 15 pour cent en poids, et de manière encore davantage préférée d'environ 3 à environ 12 pour cent en poids, du produit de réaction sur la base du poids total de la composition.

13. Composition selon la revendication 12, dans laquelle la composition comprend d'environ 5 à environ 10 pour cent en poids du produit de réaction, sur la base du poids total de la composition.

14. Composition selon l'une quelconque des revendications précédentes, comprenant en outre un excipient.

15. Utilisation de la composition comme définie dans l'une quelconque des revendications 1 à 14 dans un produit cosmétique.

16. Utilisation selon la revendication 15, dans laquelle le produit cosmétique est un agent purificateur, un stick anti-transpirant, un stick déodorant, un écran solaire, un purificateur facial, un démaquillant, une soin pour les cheveux, un gel facial, un humidifiant huile en eau, une lotion, un conditionneur, un shampooing, un shampooing conditionneur, un dentifrice ou un gel de lavage moussant pour le corps.

17. Formulation thérapeutique à utiliser sur les humains, les animaux ou les plantes, comprenant la composition selon l'une quelconque des revendications 1 à 14 et un excipient acceptable.

18. Procédé d'amélioration de la biodisponibilité de tocophérol comprenant l'étape consistant à faire réagir :
(a) un ou plusieurs dérivés de phosphate de tocophérol ; avec
(b) un ou plusieurs des agents complexants définis dans l'une quelconque des revendications 1 à 4.
